# EUROPEAN PATENT APPLICATION

(11) **EP 3 848 938 A1**
(43) Date of publication of application: **14.07.2021**
(21) Application number: 20290003.1
(22) Date of filing: 10.01.2020
(51) Int. Cl.: G16H 20/30, G16H 40/60, G16H 50/50

(54) **SYSTEM AND METHOD FOR CONFIGURING A MEDICAL DEVICE OR PATIENT TREATMENT OPTION**

(71) Applicant: Koninklijke Philips N.V., 5656 AG Eindhoven (NL)
(72) Inventor: Cox, Lieke Gertruda Elisabeth, 5656 AE Eindhoven (NL); Lavezzo, Valentina, 5656 AE Eindhoven (NL); Bulut, Murtaza, 5656 AE Eindhoven (NL); Hendriks, Cornelis Petrus, 5656 AE Eindhoven (NL); Morales, Hernan, 5656 AE Eindhoven (NL); Van der Sluis, Olaf, 5656 AE Eindhoven (NL)
(74) Representative: de Haan, Poul Erik

(57) **Abstract**

A system (10) is for configuring operation settings of a medical device (16) in a state of interaction with a patient (14), or determining features or options of a patient treatment, based on use of co-operative feedback between digital models (34, 32) for the medical device (16) on the one hand and at least a portion of the anatomy with which the medical device is interaction on the other. A feedback loop is implemented between outputs (46, 48) and inputs (42, 44) of the two models, so that outputs of each model form at least part of the input provided to the other of the models. This way a decision outcome regarding optimum operation settings for the medical device, for balancing both medical aims and also operational efficiency aims for the medical device, can effectively be arrived at iteratively through recursive circular feedback between the two models. The decision outcome may be refined though this process of back and forth feedback. At least one complete loop is implemented between the two models to determine at least one decision outcome.

## Description

### FIELD OF THE INVENTION

This invention relates to a system and method for configuring operation settings of a medical device or determining patient treatment options, in particular based on use of digital twin models for the device and a patient with which the device is in a state of physical interaction.

### BACKGROUND OF THE INVENTION

A recent development in technology is the so-called digital twin concept. In this concept, a digital representation (the digital twin) of a physical system is provided and connected to its physical counterpart, for example through the Internet of things as explained in US 2017/286572 A1. Through this connection, the digital twin typically receives data pertaining to the state of the physical system, such as sensor readings or the like, based on which the digital twin can predict the actual or future status of the physical system, e.g. through simulation, as well as analyze or interpret a status history of the physical twin. It essentially provides a digital replica of the physical object, which permits for example monitoring and testing of the physical object without needing to be in close proximity to it. In case of electromechanical systems, this for example may be used to predict the end-of-life of components of the system, thereby reducing the risk of component failure as timely replacement of the component may be arranged based on its end-of-life as estimated by the digital twin.

Digital twins are most typically used to represent mechanical or electrical devices such as manufacturing machines or even aircraft. Such digital twins are useful to monitor functioning of a device and schedule maintenance for example.

Such digital twin technology is also becoming of interest in the medical field, as it provides an approach to more efficient medical care provision. For example, the digital twin may be built using imaging data of the patient, e.g. a patient suffering from a diagnosed medical condition as captured in the imaging data, as for instance is explained by Dr Vanessa Diaz in https://www.wareable.com/health-and-wellbeing/doctor-virtual-twin-digital-patient-ucl-887 as retrieved from the Internet on 29 October 2019. Such a digital twin may serve a number of purposes. Firstly, the digital twin rather than the patient may be subjected to a number of virtual tests, e.g. treatment plans, to determine which treatment plan is most likely to be successful to the patient. This therefore reduces the number of tests that physically need to be performed on the actual patient.

The digital twin of the patient for instance further may be used to predict the onset, treatment or development of such medical conditions of the patient using a patient-derived digital model, e.g. a digital model that has been derived from medical image data of the patient. In this manner, the medical status of a patient may be monitored without the routine involvement of a medical practitioner, e.g. thus avoiding periodic routine physical checks of the patient. Instead, only when the digital twin predicts a medical status of the patient indicative of the patient requiring medical attention based on the received sensor readings may the digital twin arrange for an appointment to see a medical practitioner to be made for the patient. This typically leads to an improvement in the medical care of the patient, as the onset of certain diseases or medical conditions may be predicted with the digital twin, such that the patient can be treated accordingly at an early stage, which not only is beneficial to the patient but can also reduce (treatment) costs. Moreover, major medical incidents that the patient may be about to suffer may be predicted by the digital twin based on the monitoring of the patient's sensor readings, thereby reducing the risk of such incidents actually occurring. Such prevention avoids the need for the provision of substantial aftercare following such a major medical incident, which also alleviates the pressure on a healthcare system otherwise providing such aftercare.

In addition to representing patients, digital twins may also be used to represent medical devices and equipment.

One class of medical device which finds advantageous use with digital twins are medical devices which are designed to be in a state of continuous or regular physical interaction with a patient. These may include for example implantable devices which may have configurable operation settings, and designed to perform a particular function or task in relation to the patient's body. Examples of such medical devices include for instance ventricular assist devices, artificial organs, stents, pacemakers, mechanical ventilators, heart-lung machines, dialysis machines. Digital twins can be useful for such devices for modelling a live state of the device and its interaction with the patient as well as for generating predictions as regards device lifetime, device wear and tear, power level, and for predicting optimum operation settings for meeting certain specified target outcomes, such as minimizing wear or maximizing device lifetime.

Selecting optimum operation settings for such medical devices with a view to meeting the medical needs for the patient while also balancing operational efficiency of the device (to maximize lifetime, reduce wear) remains a challenge. Also determining optimum parameters of a patient treatment for meeting medical needs of a patient in view of constraints or requirements of a medical device is also a challenge.

### SUMMARY OF THE INVENTION

Developments in this field are generally sought. The invention is defined by the independent claims. The dependent claims define advantageous embodiments.

According to examples in accordance with an aspect of the invention, there is provided a system for configuring operation settings of a medical device and/or determining parameters of a patient treatment, the medical device adapted in use to be in a state of physical interaction with a patient, the system comprising:
a data storage arrangement storing
   a first digital model of at least a portion of an anatomy of the patient, configured to receive one or more model inputs and to simulate an actual physical state of said at least part of the anatomy based on the inputs, and to generate one or more model outputs relating to a current or future state of the anatomy;
   a second digital model of the medical device, operable to receive one or more model inputs and to simulate an operational state of the medical device based on the model inputs, and to generate one or more model outputs including one or more pertaining to proposed operation settings for the device;
a processing arrangement communicable with the data storage arrangement to access the stored digital models,
wherein the processing arrangement is configured to implement a feedback loop between the outputs of each of the digital models and the input of the other of the digital models,
wherein at least one decision outcome about a device operation setting or a patient treatment action or option or parameter is made based on running at least one complete loop.

A feedback loop in this context means that the at least one model output of the second model is provided as a model input for the first model and the at least one model output of the first model (e.g. based on the received input from the second model) is provided as a model input of the second model. The loop may start at either the first digital model or the second digital model. One loop is completed once the second model has generated a new, updated model output based on the received input from the first model for example. At this point, the loop has arrived back at the beginning.

The digital models are for example digital twins of the patient and of the medical device respectively, i.e. they simulate an ongoing state of their respective systems and provide a live replica of the system (patient anatomy or medical device).

Embodiments of the invention are based on implementing co-operative bi-directional feedback between digital twins of a medical device and a patient with which the medical device is in a state of physical interaction. For devices in a state of physical interaction with a patient, the state of the medical device directly affects the physical state of the patient. Hence, the outputs of the medical device digital twin (regarding for instance its operational or physical status) are directly relevant to modelling the real-time state of the patient and of predicting future states of the patient (based for instance on future predicted state of the device from the device digital twin). Hence prediction outputs of the device digital twin can be a useful input to the patient digital twin as it provides information which can be used to supplement any physical sensor data pertaining to the patient to assist in accurately updating the (simulations of) the patient digital twin.

Likewise, the configuration of the device may typically be adjusted at least partially in dependence upon the state of the patient, and the predicted effect of changes to the device operation on the physical state of the patient. Hence, outputs from a patient digital twin can be a useful input to the device digital twin for use in adjusting current and future settings of the device.

Furthermore, embodiments of the invention in particular propose implementing a feedback loop between outputs of each digital twin and the input of the other digital twin. In other words, outputs of each respective digital twin are provided as inputs to the other of the digital twins. This reciprocal feedback between outputs and inputs of the two digital twins incorporates at least one complete loop, e.g. from the output of the first model to the input of the second model and then back again from the output of the second model to the input of the first model. The loop can however start at either digital model. This allows the digital models to refine the decisions about the device settings or patient treatment options. In other words, decisions about settings for the device or patient treatment are effectively arrived at iteratively via recursive back and forth feedback between the digital twin of the patient and the digital twin of the device. In other words, they pass their respective prediction results back and forth between one another (at least once, and preferably a number of times) with the aim of converging co-operatively toward an optimum decision (e.g. based on balancing a number of outcome aims, both medical aims for the patient and also device aims, e.g. maximizing device lifetime or power).

In an advantageous set of examples for instance, the second digital model is configured to receive at least one model input indicative of a target change to a physical state of the at least portion of the anatomy of the patient, and to generate at least one model output indicative of an operation setting of the device for achieving said target change and a resulting change to the state of interaction between the device and patient.

Furthermore, additionally or alternatively, the first digital model may be configured to receive at least one model input indicative of a proposed change to a state of interaction between the device and patient, and to generate at least one model output indicative of a predicted change to a physical state of the at least portion of the anatomy of the patient resulting from the proposed change to the interaction state.

In this way, the model inputs and model outputs of the two digital models reciprocally co-operate, such that the at least one output of the second model can form at least one input of the first and vice versa. In this way a feedback loop about the two models can be implemented.

Preferably, the first digital model is also configured to generate prediction outputs indicative of an optimum required change to a physical state of the patient to achieve a particular medical aim or outcome. This can be generated initially and provided to the second digital model as a model input for example. Then the first model may receive back from the second model the proposed change to the device interaction state in order to achieve the change to the physical state of the patient. The first model may then predict (more accurately) what would be the effect on the physical state of the patient resultant from said proposed change to the device-patient interaction state.

Based on the result of this (and comparison with its initially calculated intended change), it may adjust the initially predicted required change to the patient physical state to achieve the medical aim. This adjusted physical change requirement may then be passed back to the second digital model, and so on.

Thus, the decision outcome can be iteratively adjusted based on back and forth or circular feedback about the two models, to converge gradually toward an optimum decision which for example balances the medical aim with the device optimization parameters.

Preferably, the processing arrangement is further configured to implement the at least one decision outcome (once made) by controlling the device to adjust the relevant operation setting based on the decision outcome. It may be configured to communicate the decision outcome to the medical device. It may generate a control command indicative of the decided operation setting change to the medical device for example.

In the case that the decision relates to a parameter of a patient treatment, the processing arrangement may be configured to control a further patient treatment device arranged in communication with the processing arrangement to implement the treatment option or action. Alternatively, the determined treatment option, action or parameter may be output to a user interface unit for communication to a user such as a clinician or the patient.

In preferred embodiments, the processing arrangement is arranged in use for receiving sensor data from one or more patient sensors and/or device sensors, and is configured to update one or both of the digital models based on the received sensor data. In this way, the digital models are kept an up-to-date accurate replica of the patient anatomy and operational state of the medical device respectively. Patient sensors may transfer to the system sensor data indicative of for example vital signs such as heart rate, blood pressure, SpO₂. The patient sensors may also include one or more imaging sensors or devices such as ultrasound sensors or scanners. The device sensors may be for monitoring key operational indicators of the device such as temperature, power usage, orientation, as well as parameters specific to the function of the device, e.g. drug delivery rate or electrical stimulation level.

Preferably at least the patient digital model is updated based on patient sensor data so that the patient digital model is kept an accurate replica of the physical state of the at least portion of the patient's anatomy.

According to one or more embodiments, the system may include one or more patient sensors and/or device sensors, arranged communicatively coupled with the processing arrangement.

In advantageous embodiments, the processing arrangement may be configured in use to recurrently or continuously update one or both of the digital models with sensor data.

Preferably, the processing arrangement is configured to recurrently or continuously update one or both of the digital models during running of the feedback loop. Thus, in some examples, the system is arranged for example to update one or both of the digital models recurrently or continuously during use of the system, for example during an operation period of the system, when the models are being actively run to generate outputs, e.g. during the running of the feedback loop.

Preferably at least the patient digital model is updated continuously or recurrently so that the patient digital model is maintained as an accurate real-time (live) replica of the physical state of the at least portion of the patient's anatomy.

According to one or more embodiments, the at least one decision outcome may be made based on running a plurality of complete loops between the two digital models. The processing arrangement may be configured to make the decision outcome based on a pre-defined set number of loops, or it may be configured to make the decision based on reaching a pre-defined similarity or proximity between a certain characteristic of outputs of one or both of the models.

According to advantageous embodiments, the feedback loop may be run continuously during operation of the system. For example, it may be run continuously over a given operation period of the system.

In advantageous embodiments, the decision outcome may be recurrently or continuously updated during running of the feedback loop. Preferably each updated decision outcome may then be implemented accordingly by adjusting the device operation setting or controlling a patient treatment device to implement the patient treatment action or in accordance with the treatment parameter.

According to one or more embodiments, the system may include the medical device. Alternatively, the medical device may be external to the system, with the system arranged to be communicative with the medical device in use.

Non-limiting examples of medical devices which may be used with or in accordance with the system include for instance: a ventricular assist device; an arterial stent; a deep brain stimulation electrode (for treatment of Parkinson's disease).

The system may be particularly advantageous for application with implantable devices since these are in a continuous state of physical interaction with the patient. Non-limiting examples of such devices include for instance: ventricular assist devices, artificial organs, stents, pacemakers, knee/hip replacements. The medical device may alternatively be a non-implantable device. Non-limiting examples of non-implantable medical devices include for instance: mechanical ventilators, heart-lung machines, dialysis machines, and robotic limbs.

Examples in accordance with a further aspect of the invention provide a method for configuring operation settings of a medical device or determining parameters of a patient treatment, the medical device adapted in use to be in a state of interaction with a patient and having operation settings for configuring the state of interaction with the patient, the method comprising:
retrieving a first digital model of at least a portion of an anatomy of the patient, configured to receive one or more model inputs and to simulate an actual physical state of said at least part of the anatomy based on the inputs, and to generate one or more model outputs relating to a current or future state of the anatomy,
retrieving a second digital model of the medical device, operable to receive one or more model inputs and to simulate an operational state of the medical device based on the model inputs, and to generate one or more model outputs including one or more pertaining to proposed operation settings for the device;
the method further comprising implementing a feedback loop running between the respective outputs of each of the digital models and input of the other of the digital models; and
making at least one decision outcome about a device operation setting or a patient treatment option or action or parameter based on running at least one complete loop of the feedback loop.

According to an advantageous set of embodiments for instance, the second digital model may be configured to receive at least one model input indicative of a target change to a physical state of the at least portion of the anatomy of the patient, and to generate at least one model output indicative of an operation setting of the device for achieving said target change and a resulting change to the state of interaction between the device and patient.

According to advantageous embodiments, the first digital model may be configured to receive at least one model input indicative of a proposed change to a state of interaction between the device and patient, and to generate at least one model output indicative of a predicted change to a physical state of the at least portion of the anatomy of the patient resulting from the proposed change to the interaction state.

Preferably, the first digital model is also configured to generate prediction outputs indicative of an optimum required change to a physical state of the patient to achieve a particular medical aim or outcome. This can be generated initially and provided to the second digital model as a model input for example.

According to one or more embodiments, the method may comprise receiving sensor data from one or more patient sensors and/or device sensors, and updating one or both of the digital models based on the received sensor data.

Preferably, one or both of the digital models may be recurrently or continuously updated with sensor data.

Preferably at least the patient digital model is updated based on patient sensor data so that the patient digital model is kept an accurate replica of the physical state of the at least portion of the patient's anatomy.

According to one or more embodiments, the system may include the one or more patient sensors or device sensors, arranged communicatively coupled with the processing arrangement.

The system may be arranged to update one or both of the digital models recurrently or continuously during use of the system, for example during an operation period of the system, when the models are being actively run to generate outputs, e.g. during the running of the feedback loop.

Preferably at least the patient digital model is updated based continuously or recurrently so that the patient digital model is maintained as an accurate real-time (live) replica of the physical state of the at least portion of the patient's anatomy.

According to one or more embodiments, the decision outcome may be recurrently or continuously updated during running of the feedback loop.

Preferably, the method further comprises implementing each updated decision outcome accordingly by adjusting the device operation setting, or adjusting operation settings of a patient treatment device, such as a medication dispenser.

In other examples, the decision outcome may be output to a user interface device, for instance presented on a display or other sensory output means for communication to a user, such as a clinician or the patient themselves.

Examples in accordance with a further aspect of the invention provide a computer program product comprising code means configured, when run on a processor, to perform the method in accordance with any example or embodiment outlined above or described below, or in accordance with any claim of this application.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a schematic diagram of interaction between components of an example system according to one or more embodiments;
Fig. 2 shows a further schematic diagram of an example system in accordance with one or more embodiments, illustrating an example feedback loop between the digital twins;
Fig. 3 schematically illustrates example communication channels which may be implemented between components of the system in accordance with one or more embodiments; and
Fig. 4 shows a block diagram of an example method in accordance with one or more embodiments.

### DETAILED DESCRIPTION OF THE EMBODIMENTS

The invention will be described with reference to the Figures. It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for configuring operation settings of a medical device in a state of interaction with a patient, or of determining features or parameters of a patient treatment, based on use of co-operative feedback between digital models for the medical device on the one hand and at least a portion of the anatomy with which the medical device is interaction on the other. A feedback loop is implemented between outputs and inputs of the two models, so an output of each model forms at least part of an input provided to the other of the models. This way a decision outcome about optimum operation settings for the medical device, for balancing both medical aims and also operational efficiency aims for the medical device, can effectively be arrived at iteratively through recursive feedback between the two models. The decision outcome may be refined though this process of back and forth feedback. At least one complete loop is implemented between the two models to determine at least one decision outcome.

Embodiments of the present invention hence aim to facilitate (preferably continuous) bidirectional functional interaction between digital twins, where functional models of the human and medical device are used to make predictions, which predictions are then used as input for the other digital twin. Thus, in embodiments, it is proposed to use digital twins for the patient and medical device to predict certain outcomes, and then to communicate these predictions from one twin to the other, such that each twin operation can be optimized not only on the native twin predictions, but also based on the partner twin predictions. In order words, data generated by a first digital twin (e.g. DT1 output) is provided as an input for a second digital twin (DT2). This is different for example from simply generating separate DT1 and DT2 outputs, and then comparing or analyzing the relations between them.

By having the digital twins of the patient and medical device directly communicate (e.g. their prediction outputs) with one another, this may help to optimize the results for both device and patient at the same time.

This maybe especially beneficial for implantable devices, i.e. devices implanted in the body, as there is continuous interaction between patient and device during prolonged periods of time, in which they mutually influence each other. However, embodiments of the invention can also be advantageously applied for use with non-implantable devices interacting with a patient's body, such as mechanical ventilators, and dialysis machines.

To better understand the proposed invention, the general motivation behind the invention and a brief discussion of existing approaches will now be outlined.

In general, in medical practice, often interactions between medical devices and patients occur, for example for diagnosis (e.g. imaging equipment), or delivering treatment (e.g. radiation therapy, surgical robot, pacemaker). The settings and operations of these devices need to be tailored to each individual patient to meet medical needs and also to fit their particular physiology or anatomy.

For example, in a standard known set-up, a medical device interacts with the body of a patient and thereby has an effect on the patient. In turn, this interaction also has an effect on the device, for example through wear and tear.

Sensors may be present that measure patient parameters (e.g. heart rate (HR), temperature, blood pressure (BP)). These parameters may in some instances be fed back to the device, to adjust the device settings. For example, in case of a cardiac assist device for (partially) replicating the function of a patient's heart, in the case that perfusion is too low, the pump speed may be increased to increase blood flow.

In addition, sensors may be present that measure parameters of a physical or operational state of the medical device, such as temperature, pump speed, or power. These parameters may in some cases also be fed back to the medical device for use in adjusting device settings. For example, in the case that temperature is becoming too high, settings may be adjusted to reduce intensity of device activity for instance.

In addition to this interaction between the patient, device and sensors, there may also be interaction with a user, e.g. a healthcare professional (HCP). A user may for instance manually adjust device settings and read out sensor data.

To improve the tailoring of device settings to the specific patient needs, a patient digital twin might be provided. A patient digital twin for example can help as simulations can be used to predict the outcomes of various treatment choices. Similarly, the device may also be provided its own digital twin, for example to monitor device functioning and schedule maintenance.

In this regard, one might for example consider a more advanced system which comprises respective digital twins of the patient and the medical device in interaction with the patient. In such a situation, patient parameters measured by patient sensors, e.g. heart rate or temperature, may be used to update the patient digital twin. The patient digital twin may be operable for instance for predicting a risk of an infection.

In addition, one or more device sensors might be provided for measuring parameters of the device, e.g. relating to its operational state, for example temperature, rotational speed, drug delivery rate. These may be provided as an input to the device digital twin, to predict interaction with the patient anatomy, and for example to predict future device failure. The device digital twin predictions might in turn may be fed back to the medical device itself for use in adjusting the device settings, e.g. to prevent the risk of device failure or for maximizing device lifetime.

In accordance with embodiments of the present invention however, it is proposed to go one step further, and provide the digital twin of the patient and the digital twin of the device in direct interaction with each other.

For example, the patient digital twin may predict, based on a medical aim for the patient, a need for a particular change to the patient physical state. For example, the patient digital twin may predict a certain perfusion need for the patient to meet a particular aim. This perfusion need may then be provided as an input to the device digital twin. Based upon this input, the device digital twin may be able to predict which pump speed (i.e. operational setting) would be required to obtain the adequate blood flow to fulfil this perfusion need (change to the physical state) for this particular patient.

However, at the same time the device digital twin may predict what the effect of such speed would be on device wear and tear and thrombus formation due to shear stress. Means for making such prediction is outlined for example in the paper: High fidelity computational simulation of thrombus formation in Thoratec HeartMate II continuous flow ventricular assist device. Wu, WT, et al. 2016, Scientific Reports, Vol. 6.

Before adjusting the device settings, the device digital twin predicted pump speed and the consequent blood flow (i.e. predicted change to the device-patient interaction state) may be fed back as an input to the patient digital twin. Based on this input, the patient digital twin may then predict whether adequate perfusion will be achieved (on short and longer term) from such a change to the blood flow (the device-patient interaction).

Thus effectively a circular feedback loop is implemented from the patient digital twin to the device digital twin and then back again with the aim of refining the proposed operation setting for the device.

Such communication between the patient digital twin and device digital twin permits improved decisions to be made regarding adjustment of the device operational settings, since account can be taken of the current state of both the patient and device, as well as constraints or priorities of both the device (e.g. device efficiency) and the patient (e.g. medical need). Thus it is possible to determine device settings in a way that optimizes both the patient care and device care at the same time.

This may be particularly advantageous, for example in the case of implantable devices, where maximizing device lifetime is an important factor, since surgery is otherwise required to replace faulty or expired devices. By utilizing feedback loops between the digital twin of the device and the patient, the patient medical needs can be met while at the same time optimizing settings to maximize device lifetime.

For example, if by running simulations on the patient digital twin, it is predicted that the patient will be stable for the next eight hours, this may be communicated to the device digital twin which can run simulations to decide which sensors to use, or how to modify sensor settings (e.g. sampling frequency) to increase the lifetime of the device.

Similarly, if simulations run on the medical device digital twin show that some functionality of the device would be lost (e.g. some sensors stop working) in one month, then the effects of these changes on the patient can be evaluated via the patient digital twin, and corresponding actions to mitigate any ill effects on patient medical condition can be planned.

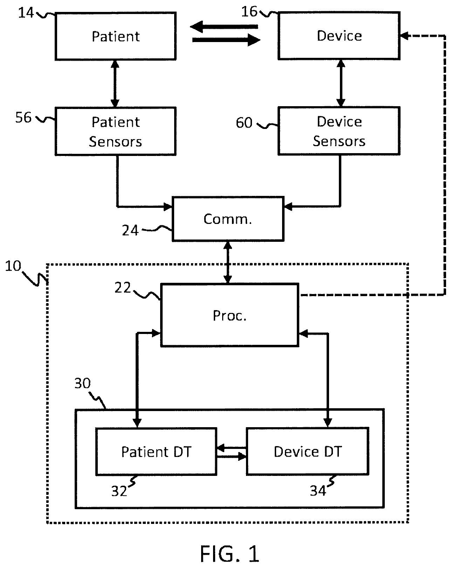

An example system in accordance with one or more embodiments is outlined schematically in Fig. 1. The system 10 is shown arranged in communication with one or more patient sensors 56 and one or more device sensors 60, via a communication module 24, for updating the patient and device digital twins 32, 34, respectively. However, this is optional and the system itself comprises at minimum the components indicated in the dashed box 10.

The system 10 is a system for configuring operation settings of a medical device 16, the medical device adapted in use to be in a state of interaction with at least a portion of the anatomy of a patient 14 and having operation settings for configuring the state of interaction with the patient.

The system comprises a data storage arrangement 30 storing a first digital model 32 ("Patient DT") of at least a portion of an anatomy of the patient and a second digital model 34 ("Device DT") of the medical device. Each model is operable to receive one or more model inputs, and to simulate an actual physical state of said at least part of the anatomy or of said medical device respectively based on the inputs, and to generate based on the inputs one or more model outputs.

More particularly, the first digital model 32 of the at least portion of an anatomy of the patient 14 is configured to receive one or more model inputs and to simulate an actual physical state of said at least part of the anatomy based on the inputs, and is configured for generating one or more model outputs relating to a current or future state of the anatomy.

The second digital model 34 of the medical device 16 is operable to receive one or more model inputs and to simulate an operational state of the medical device based on the model inputs, and is configured for generating one or more model outputs including one or more pertaining to a proposed operating setting for the medical device.

The system further includes a processing arrangement 22 communicable with the data storage arrangement 30 to access the stored digital models 32, 34.

In use, the processing arrangement is configured to implement a feedback loop running between the respective outputs of each of the digital models 32 and input of the other of the digital models 34. At least one decision outcome 50 about a device 16 operation setting is made based on running at least one complete loop.

As shown, the processing arrangement is preferably further arranged to be communicable in use with the medical device 16. The processing arrangement is preferably configured in use to communicate the decision outcome 50 made about the device operation setting to the medical device. The processing arrangement thus implements the decision by causing the medical device 16 to adjust its operation setting according to the decision outcome.

As mentioned, the processing arrangement 22 is preferably further arranged to be communicatively coupled in use with a set of one or more patient sensors 56 for providing patient sensor data for updating the patient digital model 32. It is preferably also arranged in use to be in communication with a set of one or more medical device sensors 60 for receiving sensor data pertaining to an operational state of the medical device for updating the medical device digital twin 34. As illustrated in Fig. 1, the sensors 56, 60 may in some examples be communicatively coupled to the processing arrangement via an intermediary communication module 24 which provides a communication interface. This may for instance comprise a wired or wireless communication link for communicatively linking the sensors and processing arrangement 22. It may comprise a local area network, an Internet connection, or any other form of connection or link. The processing arrangement 22 may also be communicatively coupled in use with the medical device via the communication module 24 in some examples.

The system is arranged to recurrently or continuously update or develop each of the patient 32 and device 34 digital models based on the sensor data received from the patient 56 and device 60 sensors respectively in order to keep the models as up-to-date live replicas or representations of the real-time physical and operation state respectively of the patient (anatomy) and the medical device.

The processor arrangement 22 of the computer system 20 may take any suitable shape. The processor arrangement may for example comprise one or more processors, processor cores or the like that cooperate to form such a processor arrangement. It may consist of a single component, or its functions may be distributed among a plurality of processing components.

Similarly, the communication module 24 may take any suitable shape, such as a wireless or wired data communication module, as is well known in the art and will therefore not be further explained for the sake of brevity only. Furthermore, although in Fig. 1, the communication module is shown as a separate component, this is merely schematic, and the communication module may be merely a functional module. Its function may be performed by a separate component, or its function may be performed by the processor arrangement itself or by another component of the system.

The digital models 32, 34 in the remainder of this application may also be referred to respectively as a digital twins of the patient 14 and of the medical device 16.

With regards to the patient digital twin 32, such a digital twin typically provides a model of both the elements and the dynamics of the at least portion of the anatomy of the patient (i.e. the physical twin). The digital twin may by way of example integrate artificial intelligence, machine learning and/or software analytics with spatial network graphs to create a 'living' digital simulation model of the at least portion of the patient's anatomy. By way of non-limiting example, the at least portion of the patient's anatomy may be a part of a lumen system of the patient (e.g. vascular or digestive systems), such that the digital twin comprises a model of this part of a lumen system of the patient 14. Such a living digital simulation may for example involve the use of a fluid dynamics model, a systemic model, a tissue deformation model and/or a fluid-structure interaction model in order to develop or update the digital twin based on received sensor data indicative of parameters of a physical state of the patient.

In other words, the sensor data provided by the one or more patient sensors 56 may be used to update and change the digital twin dynamically, and in real time, such that any changes to the patient 14 as highlighted by the sensor data are reflected in the digital twin. As such, the digital twin may in some examples form a learning system that learns from itself using the sensor data provided by the one or more sensors 56. The digital twin may in some cases thus be a dynamic model which dynamically develops or updates so as to provide an accurate representation of the patient's real anatomy.

The digital twin 32 typically encompasses one or more biophysical models which are used to model the anatomy represented by the model.

The digital twin 32, of the patient 14 may be initially developed from patient data, e.g. imaging data such as CT images, MRI images, ultrasound images, and so on. A typical workflow for creating and validating a 3D, subject-specific biophysical model is depicted in "Current progress in patient-specific modeling", by Neal and Kerckhoff, 1, 2009, Vol. 2, pp. 111-126. For example, in case of a digital twin representing part of the cardiovascular system of the patient 14, such a biophysical model may be derived from one or more angiograms of the patient. For example, the sensor data produced by the patient sensors 56 may be used to continuously or periodically update the boundary condition of a flow simulation through the digital lumen model (i.e. the digital twin) of the patient 14.

In operation, the processor arrangement 22 may develop or update the digital twin 32 using the received patient sensor 56 data in order to simulate the actual physical state of the at least portion of the anatomy of the patient 14.

Development and implementation of digital twin models for various example applications are described in the literature for this field. By way of example, implementation details for various example digital twin models are described in the following papers: Gonzalez, D., Cueto, E. & Chinesta, F. Ann Biomed Eng (2016) 44: 35; Ritesh R. Rama & Sebastian Skatulla, Towards real-time cardiac mechanics modelling with patient-specific heart anatomies, Computer Methods in Applied Mechanics and Engineering (2018) 328; 47-74; Hoekstra, A, et al, Virtual physiological human 2016: translating the virtual physiological human to the clinic, interface Focus 8: 20170067; and "Current progress in patient-specific modeling", by Neal and Kerckhoff, 1, 2009, Vol. 2, pp. 111-126. Details are also outlined in "Computational Biomechanics for Medicine", Grand R. Joldes et al, Springer.

In general, the digital model, e.g. of an organ or tissue area of the patient, incorporates a number of different (e.g. heterogeneous) material properties as parameters of the model, which may include blood vessels, muscles, fat, lining tissue, bones, calcified areas, which each have specific (biomechanical) material properties. These material properties form parameters for the model which can be developed based on input data such that the model provides an accurate reflection of the state of the anatomy.

In some cases, the model may be configured to permit the interaction between the tissue and the medical device for instance to be simulated.

The fundamentals of a patient-specific digital model for a given patient's anatomy may be developed in advance of operation of the system 10, such that before the system is activated, the patient digital model 32 is an accurate representation of the current physical state of the portion of the anatomy of the patient to be operated on, and incorporates sufficient information and knowledge about the material properties and physical response characteristics to allow the model to be dynamically evolved or developed or updated during operation for instance based on received patient sensor 56 data.

The parameter values may be obtained from literature and mapped onto the model, or obtained directly from measurements, e.g. elastography, performed on the patient 14.

Similar principles may also be applied for creating or developing the second digital twin 34 (for the medical device). This typically also comprises one or more computational models and may optionally also incorporate one or more machine learning algorithms to permit the model to be a self-learning system which learns from past development behavior of the medical device and uses this to improve predictions regarding future development or evolution of the device operation state. As with the patient digital twin, the medical device digital twin models a current state of the medical device and permits the execution of simulations based on the simulated current operational state for predicting future development of the operational state. These simulations also permit prediction of the effect on device-patient interaction of certain changes to the operation state. Thus based on execution of simulations, the second digital twin 34 is able to determine a predicted optimum operational setting change for achieving a particular change to a physical state of the patient.

As discussed above, system 10 implements a feedback loop between the patient 32 and device 34 digital twins for arriving at a decision outcome 50 regarding an operational setting of the medical device 16.

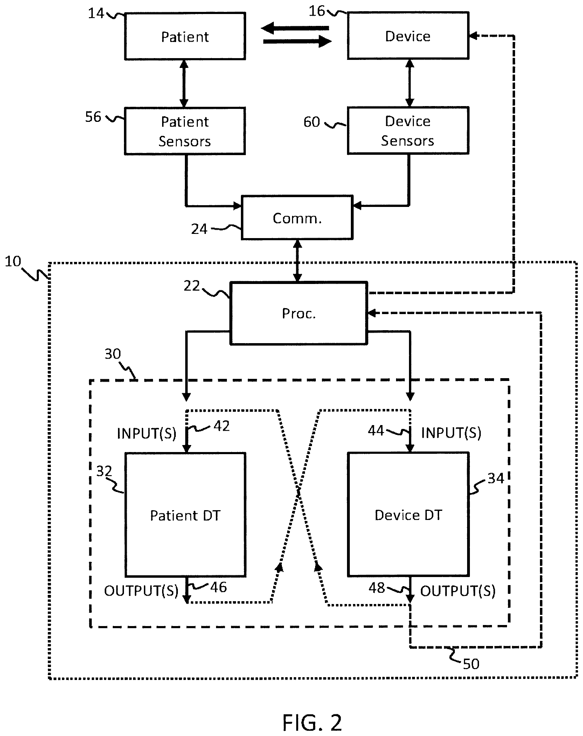

Fig. 2 shows the system of Fig. 1 with the control loop outlined more clearly.

As illustrated, the patient digital model 32 is configured to receive one or more model inputs 42, for example parameters pertaining to a physical state of the at least portion of the anatomy of the patient 14. The patient digital twin is operable to simulate an actual physical state of the patient anatomy based at least in part on received inputs. The patient digital twin is also configured for providing one or more model outputs 46, for example relating to one or more physiological or anatomical parameters or properties of the patient anatomy calculated by the model, which may be current parameters or predicted future parameters. They may be parameters which are not easily directly measurable using sensors.

The device digital twin 34 is also configured to receive one or more model inputs 44, for instance relating to an operational or physical state of the medical device 16, and to generate one or more model outputs 48 for instance relating to one or more calculated parameters pertaining to the current or predicted future operational state of the medical device 16.

As shown in Fig. 2, the feedback loop is implemented such that at least one model output 46 of the patient digital twin 32 is provided as at least one model input 44 to the device digital twin 34. Further, at least one model output of the device digital twin 48, generated based at least in part on the received input from the patient digital twin, is also provided as at least one model input 42 to the patient digital twin 32. The patient digital twin is then configured to generate at least one further model output based at least in part on the received model input from the device digital twin 34. At this point one complete loop has been run. More than one loop may be run in reaching a given decision outcome.

The feedback loop is implemented for the purpose of arriving at an optimum determination of operating settings of the medical device for the purpose of balancing both the particular medical needs of the patient and also the operational constraints or preferences (e.g. efficiency) relating to the medical device.

The medical device is designed in use to be in a state of physical interaction with the patient. This is schematically shown in Fig. 2 by the dual directional arrows between the patient 14 and the device 16. The device has an effect on the patient, but also the patient has a physical effect on the device. Depending upon operational settings of the medical device, parameters and characteristics of that device-patient interaction state can alter.

Depending upon the characteristics of the interaction state between the device and patient, a physical (e.g. anatomical or physiological) state of the patient will evolve or change at a different rate or in a different way. For example, depending upon a pump speed of a perfusion device, a patient blood flow may vary. In addition, depending upon characteristics of the interaction state a physical state of the device may evolve or change in a different way. For example, insufficient anticoagulation therapy may cause thrombosis in a ventricular assist device, which can severely affect device function.

Furthermore, depending upon the change to the physical state of the patient, a particular medical aim will either be more closely achieved, or will be moved further away from, i.e. a medical status of the patient may change.

Furthermore, depending upon the device operational setting, efficiency parameters of the device may change, e.g. wear and tear, estimated device lifetime, battery consumption etc.

Taking the above factors into account, in an advantageous set of embodiments, the digital twins and the feedback loop between them may be implemented generally as follows.

The first digital model 32 may be operable to generate a prediction output 46 indicative of an optimum required change to a physical state of the patient 14 to achieve a particular medical aim or outcome. The medical aim or outcome might be specified by a user, e.g. clinician, and may be input to the digital twin as a model input 42. The prediction output might be generated by the patient digital twin 32 as an initial step.

This model output 46 of the first digital twin 32 indicative of the target change to the physical state of the at least portion of the anatomy of the patient may then be provided as at least one model input 44 to the device digital twin 44.

The device digital twin 34 may be configured or operable to then generate at least one model output 48 indicative of an operation setting of the medical device 16 for achieving said target change to the patient physical state and also indicative of a predicted resulting change to the state of interaction between the device and patient (resulting from that change to the operational setting). The device digital twin may be configured to determine this output in accordance with one or more pre-determined model constraints or parameters. For example, model constraints may include achieving of a target device power efficiency, and/or a target rate of wear and tear or a target device lifetime.

This model output 48 indicative of the proposed change to the operation setting and resulting change to the state of interaction between the device and patient may then be provided back to the patient digital twin 32 as a model input 42 to the digital twin.

The patient digital twin 32 may then be further configured to generate, based at least in part on this input, at least one model output 46 indicative of a predicted change to a physical state of the at least portion of the anatomy of the patient resulting from the proposed change to the interaction state. The prediction in this regard of the patient digital twin may be expected to be more accurate than that of the device digital twin since the patient digital twin has been developed based on the data specific to the patient under consideration, and so is personalized to the patient anatomy and physiology. By contrast it might be that any biophysical model parts of the device digital twin are generic.

The patient digital twin 32 may compare the resulting predicted change to the physical state of the patient (resulting from the proposed operation setting change of the device digital twin 34) with the initially generated target change to the physical state. Depending upon a degree of similarity or disparity between the two, the patient digital twin may adjust or alter the target change to the patient physical state and provide this as a new model output 46 back to the input 44 of the device digital twin for re-assessment. Furthermore, based on the result of the patient digital twin, the device digital twin might also adjust (e.g. weaken) one or more of its model constraints so as to move closer toward the target medical aim.

Different weightings could be applied to medical aims versus device operational aims, e.g. a higher weighting to medical aims.

In this way, the model inputs and model outputs of the two digital models reciprocally co-operate to refine the decision outcome regarding the operation setting of the medical device.

According to one or more embodiments, additionally or alternatively to determining a proposed change to a device operation setting, the device digital twin 34 may determine, based on the model input(s) from the patient digital twin, one or more proposed changes to parameters of a patient treatment, or one or more proposed treatment actions or options or features for the patient. For example, depending upon certain parameters of other auxiliary treatment to the patient, a physical state of the medical device may change. The medical device digital twin may be configured, based on the received input indicative of the target change to the physical state of the at least portion of the anatomy, to determine a proposed treatment action or change to a parameter of a patient treatment for at least in part attaining the target change to the anatomy state, while optimizing conditions for optimal operational efficiency and longevity of the medical device. This may be instead of or in addition to determining a proposed change to a device operation setting. The final outcome of the loop in this case may be a decision outcome regarding a parameter of a treatment of a patient or a patient treatment action. This may or may not be in combination with a decision outcome as to an operation setting for the device.

By way of example, in case of an implanted device such as a stent or assist device, the level of anticoagulation therapy can affect the physical state of the device. For example, insufficient anticoagulation therapy may cause thrombosis in a ventricular assist device, which can severely affect device function. By running device digital twin simulations, with input from device sensors as well as the patient digital twin, it may be determined by the device digital twin 34 whether thrombosis is likely, now or in the future, to occur. Based on this, the device digital twin may determine a proposed adjustment to the anticoagulation therapy for avoiding thrombosis in the ventricular assist device.

This proposal may then be provided as a model input to the patient digital twin, and the patient digital twin may then be further configured to generate, based at least in part on this input, at least one model output 46 indicative of a predicted change to a physical state of the at least portion of the anatomy of the patient resulting from the proposed change to the interaction state.

The patient digital twin 32 may compare the resulting predicted change to the physical state of the patient (resulting from the proposed treatment parameter or option change or proposed treatment action from the device digital twin 34) with the initially generated target change to the physical state. Depending upon a degree of similarity or disparity between the two, the patient digital twin may adjust or alter the target change to the patient physical state and provide this as a new model output 46 back to the input 44 of the device digital twin for re-assessment. Furthermore, based on the result of the patient digital twin, the device digital twin might also adjust (e.g. weaken) one or more of its model constraints so as to move closer toward the target medical aim.

Thus it can be seen that the system may be configured to use the feedback loop between the device and patient digital models to determine decision outcomes regarding optimum device operation settings and/or changes to features or parameters of other aspects of patient treatment.

The processing arrangement 22 may be configured in use to recurrently or continuously update one or both of the digital models with sensor data. This may be done during the running of the feedback loop such that even as the digital twins co-operatively refine and move toward the decision outcome, the digital twins are continuously or recurrently kept up-to-date with a real-time state of the patient and the medical device.

The feedback loop is preferably run continuously during operation of the system.

In some examples, the system may be configurable in an idle state in which the feedback loop is not run and in which outputs from the digital models 32, 34 are not being generated, and an operational state in which the feedback loop is running, and outputs are being generated from the two digital models 32, 34. Preferably the feedback loop is run continuously during the operational state.

The decision outcome may be recurrently or continuously updated during running of the feedback loop (e.g. during continuous running of the feedback loop), and preferably wherein each updated decision outcome is implemented accordingly by adjusting the device operation setting or controlling a communicatively coupled or associated patient treatment device in accordance with the determined treatment parameters, option or action. In this way, the operation settings of the medical device or treatment of the patient are continually being updated in accordance with refinement of the decision outcome and in accordance with the changing state of the patient anatomy as the operation settings change.

The decision outcome, or each decision outcome, once made, may be output to the processing arrangement 22, which may then implement the decision, for example by adjusting the operation setting of the medical device 16 accordingly (or for instance adjusting settings of a patient treatment device to change a parameter of patient treatment). If the feedback loop is run continuously, the processing arrangement may simply query the device digital twin 34 at regular intervals for a readout of the current value or state of the determined operation parameter or treatment option and may then take this as a decision outcome for implementing at the medical device 16 or other treatment device. In further examples, the device digital twin may be arranged to communicate directly with the medical device and may output the decision outcome regarding the operation setting directly to the medical device.

In other examples, the decision outcome may additionally or alternatively be output to a user interface device for communication to a user such a clinician of the patient themselves. The user may use the communicated information to, for example, alter the patient status. In the case that the user is a caregiver of the patient, then they may decide for example to change a setting of the medical device to run a new test, or change an aspect of the patient treatment. In the case that the user is the same person as the patient (i.e. the patient is the user) and the decision outcome is given as feedback to the patient, then the patient may in some examples change their behavior based on the feedback. For instance, if the decision outcome relates to a proposed change to patient treatment, for example to a proposed change to patient activity level or supplement dosage, the patient may respond by increasing their activity level or changing their supplements.

According to one or more examples, the output from the feedback loop between the digital twins may be communicated to a further device (which is different than the medical device 16 to which the second digital twin 34 pertains) that is being used by the patient. This may be a patient treatment device for example. For example, the patient treatment device may be a medication dispenser. A decision outcome indicative of a proposed change to medication dosage might be output to the medication dispenser and the device may respond by generating an alert and dispensing a medication.

In accordance with one or more examples, the decision outcome from the feedback loop between the digital twins may be communicated to a further system, such as for instance an environment control system, which can change the context or environment parameters of the patient. For instance, based on a decision outcome regarding required change to patient condition, environmental temperature might be decreased, and oxygen content increased. By way of a further example, based on the decision output from the feedback loop, the particular set of measurements and sensor data being collected for the patient may be adjusted. This in turn may influence how the electronic health record data is generated, collected and/or structured, and/or how patient related reports are generated.

Although Fig. 2 above shows direct communication between only a certain subset of the various components, in further examples, there may be communication channels or paths (either direct or indirect) between a greater number of the components.

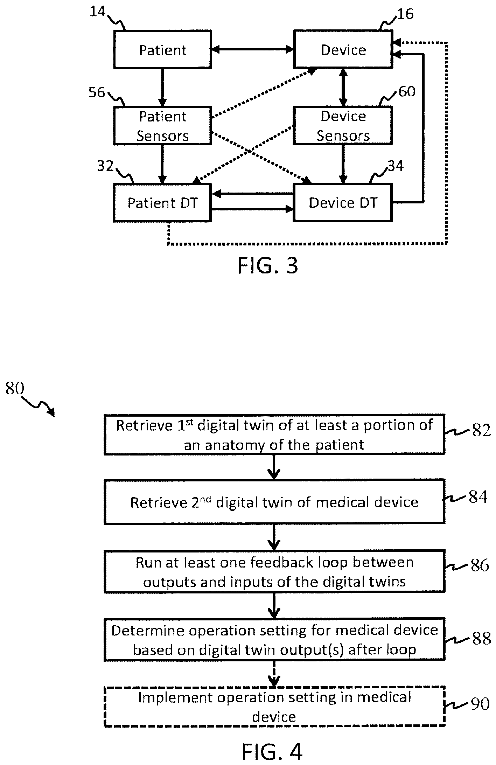

Fig. 3 schematically illustrates example communication paths or channels which might be implemented in accordance with one or more embodiments, to enhance decision making. The shown communication paths or channels may be implemented as direct communication channels or may be provided indirectly, e.g. via a communication module 24 or the processing arrangement 22 in some examples.

As illustrated in Fig. 3, in addition to bi-directional feedback between the two digital models 32, 34, the patient 56 and device 60 sensors may be arranged in some examples to communicate sensor data directly to the patient and device digital twins respectively for updating the digital twins.

Furthermore, the device sensors 60 may be arranged to communicate device sensor data to the patient digital twin. For example, device current operational parameters (e.g. pump flow) may be used as an input the patient digital twin to enable more accurate predictions about the patient physical state, e.g. of blood flow.

Furthermore, the patient sensors 56 may be may communicate patient sensor data to the device digital twin 34. For example, current patient parameters (e.g. blood pressure, activity, heart rate), may be provided as an input to the device digital twin 34, e.g. to enable better prediction of the risk of device failure.

Patient sensor 56 data may also in some examples be fed directly to the medical device 16. For example, current patient parameters may be provided to the medical device which may in some examples directly lead to adjusted device settings. For example, the device may have an emergency override function whereby the settings are adjusted urgently in case of patient parameters exceeding a defined threshold, e.g. an internal defibrillator acts upon detected arrhythmia.

In some examples, patient digital twin outputs, e.g. prediction outputs regarding change to the physical state of the patient, might be provided directly to the medical device.

In accordance with one or more advantageous embodiments, the bi-directional feedback between the device and patient digital twins via the feedback loop may additionally or alternatively be used for adapting timing and scheduling of patient and device-related operations.

By way of example, the patient digital twin may be used for optimizing diagnosis and treatment of the patient via optimization of the digital twin of the device. By way of example, if the device digital twin senses that data from the device is not reliable (e.g. due to a potential problem that is predicted), it may generate an output indicative of this. This may be received by the patient digital twin and may impact the manner in which the patient digital twin simulations using the device data are used.

For example, the information extracted from the device digital twin may influence the behavior of the patient digital twin with respect to: the timing and scheduling of diagnosis and treatment of the patient; the timing and scheduling of new data collection for updating the patient or device digital twin; and/or the timing and scheduling of modification of the patient digital twin.

By way of further example, the digital twin 34 of the medical device may be used to inform optimization of the device status, settings, and/or functions, and/or may be used to inform scheduling of maintenance via optimization of the digital twin 32 of the patient. For example, if the digital twin 32 of the patient requires that the medical device is used in a certain way (e.g. for meeting patient health outcomes), which may not be optimal for longevity of the medical device according to the device digital twin 34, the needs of the patient digital twin may be favored, in order for the patient to receive the required care. In other words, a higher weighting may be placed on outputs of the patient digital twin pertaining to medical need of the patient than outputs of the device digital twin pertaining to device optimizations.

The information extracted from the patient digital twin 32 can be combined with the device digital twin to determine optimal timing and scheduling of device maintenance; optimal timing and scheduling of modification of device settings; and/or optimal timing and scheduling of modification of the device digital settings or parameters.

A wide range of different particular applications exist for advantageous implementation of embodiments of the present invention. The principles of the present invention will thus now be elucidated further by way of the following non-limiting example applications.

One advantageous example application area is use with ventricular assist devices (VADs) implanted in patients who have suffered heart failure.

In case of severe heart failure, a ventricular assist device (VAD) can be implanted to take over (part of) the function of the patient's ventricle. This can be either temporary, pending heart transplant or recovery, or as a permanent therapy. Although the technology has improved significantly over the past decades, important challenges remain with regards to long-term use. Some of the remaining challenges have been discussed for example in the paper: Left ventricular assist devices: Challenges toward sustaining long-term patient care. Schmid Daners, M, et al. 8, 2017, Annals of Biomedical Engineering, Vol. 45, pp. 1836-1851.

Four key areas for improvement include the following:
1. Decreasing the high risk of thromboembolic and bleeding events.
2. Reducing blood damage.
3. Avoiding infection and increasing freedom of movement with a transcutaneous energy transmission system.
4. Adaptation of the VAD flow to the perfusion requirements of the patient.

Especially for areas 1, 2 and 4, it may be beneficial to have a digital twin of (the cardiovascular system of) a patient communicating with a digital twin of the VAD. For example, this could assist in determining the optimal (dynamic) VAD flow (operation setting), to balance fulfilling the perfusion requirements of the patient with maintaining the risk of blood damage at an acceptable level (high shear stress is known to lead to blood damage) and the wall shear stress conditions favorable for endothelial cell adhesion to avoid inflammatory processes.

Optimizing the assist device operation settings is very complex and strongly dependent upon the individual patient, as patients will vary in their perfusion needs but also in their risk of device-related adverse events. Schmid Daners et al. in the paper cited above have suggested physiological control systems, working collaboratively with biocompatible sensor devices, to target the adaptation of the VAD flow to the perfusion requirements of the particular patient. However, while this can help in optimizing the VAD flow to perfusion needs at a specific time-point, it is not able to not take into account longer-term consequences e.g. related to blood damage and endothelial cell adhesion. Here, use of digital twins greatly enhances the optimization.

By implementing a feedback loop system between the digital twin 32 of the patient and the digital twin 34 of the VAD, the (hemodynamic) condition of the patient can be optimized, while at the same time endothelial cell adhesion and blood damage can be modelled using the shear stress distributions derived from the digital twin of the VAD. This modelling can be applied to refine the determination as to the optimum VAD operation settings for achieving the target hemodynamic condition of the patient within the constraints of the endothelial cell adhesion and blood damage requirements.

A further example application for embodiments of the invention is for configuring settings of a stent implanted in a patient for treating stenosis.

Drug-eluting stents (DES) have rapidly became the standard of care for the percutaneous treatment of symptomatic coronary artery disease. A DES is a peripheral or coronary stent (a scaffold) placed into narrowed, diseased peripheral or coronary arteries that slowly releases a drug to block cell proliferation.

However, stent fracture has emerged as a complication following DES implantation. This is now recognized as one of the contributors to in-stent restenosis, as well as perforation and stent migration and possibly also stent thrombosis. The clinical incidence of coronary stent fracture is 1% to 2% of patients. However, it is thought that rates could be much higher than this, as it has been found, after autopsy, that the incidence of stent rupture can be near 30%.

Stent fracture has also been found to occur in almost all vascular sites including the femoral, renal, carotid, iliac, and femoropopliteal arteries. The severity of the stent fracture can be classified as follows:
Grade I: Involving a single-strut fracture.
Grade II: Two or more strut fractures without deformation of the stent.
Grade III: Two or more strut fractures with deformation of the stent.
Grade IV: Multiple strut fractures with acquired transection but without gap.
Grade V: Multiple strut fractures with acquired transection with gap.

By implementing the bi-directional communication between the digital twin of the patient and the digital twin of the stent in accordance with embodiments of the invention, calcification (i.e. medical requirements) and the risk of stent rupture (device operational state) can be both taken into account in monitoring and predicting interaction between the device and the patient and the device expected lifetime.

Fracture risk can be predicted through a combination of the modelling of the two digital twins. For example, physiological parameters of the patient such as blood pressure and heart rate can be relevant to rate of degradation of the physical integrity of the device stent. Furthermore, the stent may be fitted with local sensors (with a micro-antenna) which may monitor for instance a local blood pressure at the site of the stent or blood-stent interaction forces.

By implementing a feedback loop between the digital twin of the patient and the digital twin of the stent, configuration of the stent for meeting medical requirements while also maximizing device life time can be determined. In addition, it can be more easily determined the optimal time for device replacement in the case of restenosis.

A further potential application of embodiments of the invention is for use with deep brain stimulation (DBS) devices.

DBS is a therapy for movement disorders such as Parkinson's disease, in which an electrode is implanted at a specific location in the brain to deliver an electrical current with a certain amplitude, frequency and for a particular time duration.

Typically, closed-loop systems are used to adjust the stimulation depending on recorded brain signals.

Hardware problems can occur with the equipment including for example lead fracture, coating delamination, electrode migration and limited battery lifetime. These problems can lead to malfunction and ineffective therapy. Mitigation of these problems, if they occur, requires surgery.

The underlying cause of these problems is the mechanical and electrical load on the hardware, which is determined by the patient movements and the delivered electrical current. A system comprising a digital twin of the patient and a digital twin of the implanted system would allow to predict the remaining lifetime of the device and provide an early warning for intervention so as to prevent severe complications. It would also permit the electrical current settings to be more intelligently refined so as to meet medical requirements for the therapy but while minimizing wear to the electrodes by minimizing delivered loads for example.

As discussed above, the patient digital twin and medical device digital twin are arranged to exchange outputs co-operatively to refine decisions regarding device operational settings or parameters or features of a patient treatment. In some cases therefore, the patient digital twin includes simulation and predictive algorithms which are configured to generate outputs at least partially based on the particular types of output generated by the device digital twin, i.e. is specifically configured to be functionally co-operative with the device digital twin. The same is true vice versa for the device digital twin which is configured for receiving outputs of the type generated by the device digital twin. Thus the initial development of each of the two digital twins may be performed in a way that has regard to, or takes into account, the parameters of the other of the digital twins.

According to some embodiments, the two digital twins may be developed at least partially together in a joint training or development procedure. One approach will be explained below.

As mentioned above, according to one or more embodiments, each of the digital twins may comprise or embody one or more machine learning algorithms for performing simulations or predictions pertaining to their relevant systems. This way, each digital twin can learn from results obtained from past data for a given patient or device and thus become specifically tailored to the individual physiology of a particular patient or device.

In accordance with one or more embodiments, the machine learning algorithms of the two digital twins 32, 34 may be trained together in a joint training procedure. This provides a means by which each digital twin can be optimized to the parameters and features of the other of the digital twins.

This joint training procedure involves for example the two digital twins 32, 34 interacting with each other by exchanging data, with the purpose of improving the machine-learning engines that are present in each digital twin.

To achieve this, according to one or more embodiments, the data generated by running simulations of the Patient digital twin 32 can be provided as one of the inputs (in addition to device sensor 56 data and optionally also other device features) while training the machine learning engine for the Device 16 digital twin 34. Similarly, the data generated by running simulations on the device digital twin 34 can be used as one of the inputs while training the machine learning engine of the patient digital twin 32.

According to one set of embodiments for instance, this joint training procedure maybe implemented analogously to General adversarial networks (GANs), which will be well-known to the skilled person in this field. In GANs, there are two neural networks (i.e. two machine learning engines) provided running in parallel, wherein one network generates data, and the other network evaluates the generated data in accordance with its algorithms.

In more detail, in GANs, one network generates data (this is called the generative network) and the other evaluates the data (this is called the discriminative network). The training objective of the generative network is to increase the error rate of the discriminative network, while the training objective for the discriminative network is to decrease its error rate (i.e. improve the accuracy). Implementation of this procedure for GANs is described in detail for example in the paper: Goodfellow, I. J, et al. General Adversarial Networks, 2014, arXiv. Details are also provided for example in the paper: Goodfellow, I. NIPS 2016 Tutorial: Generative Adversarial Networks, 2016, arXiv.

For embodiments of the present invention, the standing GAN procedure may be modified by having the patient digital twin 32 and device digital twin 34 alternate roles between being the generative network and the discriminative network. Hence, in this case, in one epoch or time period, the patient digital twin 32 would act as a generative network generating real data for the device digital twin 34, with the aim of decreasing the performance of the device digital twin (which acts as a discriminative network). The device digital twin 34 would need to learn the new data to improve its performance (resulting in an improved device digital twin).

Subsequently, in the next epoch or time period, the roles are reversed. The device digital twin 34 acts as a generative network generating data that for receipt and evaluation by the patient digital twin 32 (which in this epoch acts as a discriminative network). In this way, the digital twin 32 of the patient can be optimized (i.e. trained) to be a perfect match (i.e. until there is no change in accuracy) to the device digital twin 34, and similarly the device digital twin 34 can be trained to be the perfect match to the patient digital twin 32.

In accordance with any embodiment of the invention, the system may advantageously be configured such that the two digital twins 32, 34 are linked or combined in such a way that they effectively form a single unitary digital twin for both the patient anatomy and the medical device. This option will now be explained below.

In the case in particular of implantable devices, the device 16 is in a state of continuous physical interaction with the patient. Hence, in effect, the device forms a part of the physical system of the patient's anatomy. Likewise, the patient anatomy is constantly influencing the device physical state. Thus in accordance with one or more embodiments, it may be considered to combine the two digital twins 32, 34 into a single unitary digital twin, encompassing algorithms and models for modelling and simulating states of the patient 14 and the medical device 16 at the same time.

This may be done for example by generating or initially developing the two digital models together, using patient and device data together to generate both models. For example, patient and device data can be used together for generating the physical models and/or training the machine learning engines.

One advantage of this approach may be improved modelling of the effects of the patient on the device and vice versa.

Another application of the combined modelling approach might be in evaluating the individual digital twin outputs. For example, there might be provided a patient digital twin, a device digital twin, as well as a further (combined) Patient+Device digital twin. The simulation outputs may be compared between the patient digital twin and Patient+Device digital twin, and between the Device digital twin and the Patient+Device digital twin, and these comparisons (i.e. the difference or residual between outputs) used as part of an adaptive feedback loop to improve the individual patient digital twin and Device digital twin, respectively.

Embodiments of the invention may be advantageously applied in a wide range of different application areas.

By way of one set of advantageous examples, the system with feedback loop between the two digital twins may advantageously permit optimization of patient outcome and device functioning for implantable devices such as for instance: ventricular assist devices, artificial organs, stents, pacemakers, knee/hip replacements. Embodiments may also be advantageously applied for optimizing non-implantable devices interacting with the patient body, such as for instance: mechanical ventilators, heart-lung machine, dialysis machines, and robotic limbs.

Examples in accordance with a further aspect of the invention also provide a method of configuring operation settings of a medical device, the medical device adapted in use to be in a state of interaction with a patient and having operation settings for configuring the state of interaction with the patient.

A block diagram of an example method in accordance with one or more embodiments is shown in Fig. 4.

The method 80 comprises retrieving 82 a first digital model (or digital twin) of at least a portion of an anatomy of the patient. The first digital model is configured to receive one or more model inputs and to simulate an actual physical state of said at least part of the anatomy based on the inputs, and to generate one or more model outputs relating to a current or future state of the anatomy.

The method 80 further comprises retrieving 84 a second digital model (or digital twin) of the medical device. The second digital model is operable to receive one or more model inputs and to simulate an operational state of the medical device based on the model inputs, and to generate one or more model outputs including one or more pertaining to proposed operating settings for the device.

The method 80 further comprises implementing 86 a feedback loop running between the respective outputs of each of the digital models and input of the other of the digital models.

The method 80 further comprises making at least one decision outcome 88 about a device operation setting and/or a parameter or feature of a patient treatment or a treatment action based on running at least one complete loop of the feedback loop.

In advantageous embodiments, the method may then further comprise implementing 90 the decision outcome by controlling the medical device to adjust the operational setting(s) accordingly, or controlling an associated patient treatment device in accordance with the determined patient treatment action or option.

Implementation options and details for each of the above steps may be understood and interpreted in accordance with the explanations and descriptions provided above for the apparatus aspect of the present invention (i.e. the system aspect).

Any of the examples, options or embodiment features or details described above in respect of the apparatus aspect of this invention (in respect of the system) may be applied or combined or incorporated mutatis mutandis into the present method aspect of the invention.

Examples in accordance with a further aspect of the invention also provide a computer program product comprising code means configured, when run on a processor, to perform the method 80 in accordance with any embodiment outlined above or in accordance with any claim of this application.

As discussed above, embodiments of the invention make use of a processor arrangement to perform data processing. The processor arrangement may comprise one or more processors.

Such processors can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor arrangement may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A system (10) for configuring device operation settings of a medical device (16) and/or determining parameters of a patient treatment, the medical device (16) being adapted in use to be in a state of physical interaction with a patient (14), the system comprising:
a data storage arrangement (30) for storing
a first digital model (32) of at least a portion of an anatomy of the patient, configured to receive one or more first model inputs (42) and to simulate an actual physical state of said at least portion of the anatomy based on the first model inputs, and to generate one or more first model outputs (46) relating to a current or future state of the anatomy,
a second digital model (34) of the medical device, operable to receive one or more second model inputs (44) and to simulate an operational state of the medical device (16) based on the second model inputs, and for generating one or more second model outputs (48) including one or more pertaining to proposed operation settings for the medical device(16);
a processing arrangement (22) communicable with the data storage arrangement (30) to access the stored first and second digital models (32, 34),
wherein the processing arrangement (22) is configured to implement a feedback loop between an output (46, 48) of each of the first and second digital models (32, 34) and the input (44, 42) of the other of the first and second digital models(32, 34),
wherein at least one decision outcome (50) about a device operation setting or a parameter of a patient treatment is made based on running at least one complete feedback loop.

2. A system (10) according to claim 1, wherein the second digital model (34) is configured to receive at least one second model input (44) indicative of a target change to a physical state of the at least portion of the anatomy of the patient, and to generate at least one second model output (48) indicative of a device operation setting of the medical device (16) for achieving said target change and a resulting change to the state of interaction between the medical device (16) and patient (14).

3. A system (10) as claimed in claim 1 or 2, wherein the first digital model (32) is configured to receive at least one first model input (42) indicative of a proposed change to a state of interaction between the medical device (16) and patient (14), and to generate at least one first model output (46) indicative of a predicted change to a physical state of the at least portion of the anatomy of the patient resulting from the proposed change to the interaction state.

4. A system (10) according to any of claims 1-3, wherein the processing arrangement (22) is further configured to implement the at least one decision outcome (50) by controlling the medical device (16) to adjust the relevant device operation setting based on the decision outcome, or controlling a patient treatment device to implement the patient treatment parameter.

5. The system (10) according to any of claims 1-4, wherein the processing arrangement (22) is arranged in use for receiving sensor data from one or more patient sensors (56) and/or device sensors (60), and to update one or both of the first and second digital models (32, 34) based on the received sensor data.

6. The system (10) according to claim 5, wherein the processing arrangement (22) is configured in use to recurrently or continuously update one or both of the first and second digital models (32, 34) with sensor data, and
preferably wherein the processing arrangement (22) is configured to recurrently or continuously update one or both of the first and second digital models (32, 34) during running of the feedback loop.

7. The system (10) according to any of claims 1-6, wherein the at least one decision outcome (50) is made based on running a plurality of complete loops between the first and second digital models (32, 34).

8. The system (10) according at any of claims 1-7, wherein the feedback loop is run continuously during operation of the system.

9. The system (10) according to any of claims 1-8, wherein the decision outcome (50) is recurrently or continuously updated during running of the feedback loop, and preferably wherein each updated decision outcome is implemented accordingly by adjusting the medical device (16) operation setting or adjusting a setting of a further patient treatment device in communication with the system.

10. The system (10) according to any of claim 1-9, wherein the system includes the medical device (16).

11. A method (80) for configuring device operation settings of a medical device (16) or determining a patient treatment action, the medical device (16) adapted in use to be in a state of interaction with a patient (14) and having device operation settings for configuring the state of interaction with the patient (14), the method comprising:
retrieving (82) a first digital model (32) of at least a portion of an anatomy of the patient (14), configured to receive one or more first model inputs (42) and to simulate an actual physical state of said at least portion of the anatomy based on the inputs, and to generate one or more first model outputs (46) relating to a current or future state of the anatomy;
retrieving (84) a second digital model (34) of the medical device (16), operable to receive one or more second model inputs (44) and to simulate an operational state of the medical device (16) based on the second model inputs, and to generate one or more second model outputs (48) including one or more pertaining to proposed operation settings for the medical device (16);
implementing (86) a feedback loop running between the respective outputs of each of the first and second digital models (32, 34) and inputs of the other of the first and second digital models (32, 34); and
making (88) at least one decision outcome (50) about a device operation setting or a patient treatment action based on running at least one complete loop of the feedback loop.

12. The method (80) according to claim 11, wherein
the second digital model (34) is configured to receive at least one second model input (44) indicative of a target change to a physical state of the at least portion of the anatomy of the patient (14), and to generate at least one second model output (48) indicative of a device operation setting of the medical device (16) for achieving said target change and a resulting change to the state of interaction between the medical device (16) and patient (14); and/or
the first digital model (32) is configured to receive at least one first model input (42) indicative of a proposed change to a state of interaction between the medical device (16) and patient (14), and to generate at least one first model output (46) indicative of a predicted change to a physical state of the at least portion of the anatomy of the patient (14) resulting from the proposed change to the interaction state.

13. The method (80) according to claim 11 or 12, further comprising receiving sensor data from one or more patient sensors (56) and/or device sensors (60), and updating one or both of the first and second digital models (32, 34) based on the received sensor data, and
optionally wherein the one or both of the first and second digital models (32, 34) is recurrently or continuously updated with sensor data.

14. The method (80) according to any of claims 11-13, wherein the decision outcome (50) is recurrently or continuously updated during running of the feedback loops, and
preferably further comprising implementing each updated decision outcome accordingly by adjusting the medical device (16) operation setting or adjusting a setting of a further patient treatment device.

15. A computer program product comprising code means configured, when run on a processor, to perform the method (80) of any of claims 11-14.
